# EUROPÄISCHE PATENTSCHRIFT

(11) **EP 2 289 468 B1**
(45) Veröffentlichungstag und Bekanntmachung des Hinweises auf die Patenterteilung: **05.08.2015**
(21) Anmeldenummer: 10169578.1
(22) Anmeldetag: 07.06.2005
(51) Int. Cl.: A61F 2/30, A61F 2/28, A61F 2/46, A61L 27/56, A61L 27/44

(54) **Implantatkörper und Verfahren zu seiner Herstellung**
Implant body and method for manufacturing
Corps d'implant et son procédé de fabrication

(30) Priorität: 07.06.2004 DE 102004027657
(43) Veröffentlichungstag der Anmeldung: 02.03.2011
(62) Teilanmeldung aus: 05756998.0
(73) Patentinhaber: Karl Storz GmbH & Co. KG, Postfach 230 78503 Tuttlingen (DE)
(72) Erfinder: Draenert, Klaus, 81545 München (DE)
(74) Vertreter: Vossius & Partner Patentanwälte Rechtsanwälte mbB

(56) Entgegenhaltungen:
- WO-A-95/21053
- US-A1- 2002 005 600
- US-B1- 6 316 091

## Beschreibung

In der Chirurgie am Bewegungsapparat besteht seit jeher ein Bedarf, Knochendefekte nach Frakturen, nach Entfernung von Tumoren, Knochensubstanzverluste nach Entzündungen oder in Verbindung mit Knochenzysten mit Knochenmaterial oder mit einem dem Knochen ähnlichen Ersatzmaterial wieder aufzufüllen. Hierbei wurde zum Teil auch auf bovines Ersatzmaterial zurückgegriffen [Tröster SD 1993: Die Hydroxylapatitkeramik Endobon®). Eine alternative Therapiemöglichkeit für Knochendefekte. In: Venbrocks R, Salis G von (Hrsg): Jahrbuch der Orthopädie,pp 231-246. Zülpich: Biermann ] oder Material aus Korallenriffen [Irwin RB, Bernhard M, Biddinger A (2001) Coralline hydroxyapatite as bone substitute in orthopaedic oncology. Am J Orthop 30:544-550 ] im Labor aufbereitet (replaminiforme Prozesse) und eingesetzt. Beiden Materialien haftet der Makel an, dass es biologische und damit gerichtete Strukturen sind, die man nicht mehr beeinflussen kann und die kaum standardisiert herstellbar sind. Beide Werkstoffe bringen die Anisotropie in Struktur und Eigenschaften einer gewachsenen biologischen Struktur mit und sind in fast allen Fällen zu steif. Ein Verfahren für die Herstellung solch biologischen Ersatzmaterials ist in der DE-A 3903695 beschrieben. Synthetische Ersatzwerkstoffe sind zwar aus reinen Ausgangsmaterialien herstellbar, lassen jedoch eine geordnete, dem Knochen ähnliche Struktur vermissen; ein solcher synthetischer Werbtoff ist das geschäumte Ceros®; diese Werkstoffe lassen einen knöchernen Durchwuchs vermissen, da die Poren meist geschlossen sind [Dingeldein E und H Wahlig (1992) Fluoreszenzmikroskopische Untersuchungen zur knöchernen Integration von Kalziumphosphatkeramiken. In: Merck Biomaterialien (Hrsg) Endobon® und DBCS®, Darmstadt:Merck].

In DE-A-2242 867 und US-A- 38 99 556 wird ein Verfahren mit einem präformierten, formgebenden Gerüst beschrieben mit einer dichten Schüttung von Kugeln, bei dem die formgebenden Elemente von einem Lösungsmittel übergossen werden und auf diese Weise verklebt werden sollen; mit einem solchen Verfahren lässt sich jedoch kein gleichmässig poröses interkonnektierendes Material in stardardisierter Form herstellen; zu unregelmässig waren die Verklebungen und zu unkontrollierbar der Anlösungsprozess. In EP-A-0 553167 und EP-A-0204786 wurde das Problem teilweise gelöst, indem deformierbare formgebende Elemente durch Druckbeaufschlagung in Kontakt aufeinander gepresst wurden und in diesem Zustand von der Gerüst-bildenden Masse umgossen wurden, welche danach aushärtete und anschliessend von den formgebenden Elementen chemisch oder thermisch wieder befreit wurde. Die so hergestellten Implantate liesen in der Hälfte, die der Druckbeaufschlagung zugewandt war, schöne und nahezu gleichmässige Interkonnektierungen erkennen, in der von dieser abgewandten Hälfte jedoch waren zahlreiche geschlossenen Poren ausgebildet. Formgebende deformierbare und nur teilweise elastischen Elemente dämpfen die einwirkende Kraft und die deformierende Wirkung erschöpft sich durch Dämprung, sodass keine gleichmässige und durchgehende Interkonnexion erreicht wird. Rein zufällig wurde nun ein Verfahren gefunden, welches diese Phänomene vermeiden lässt und vollständig gleichmässige Verformung aller am Prozess beteiligten formgebenden Elemente erreichen lässt. Dieses Verfahren ist denkbar einfach und daher auch besonders preiswert und vollständig reproduzierbar.

Aus WO 95/21053 A, die den nächstliegenden Stand der Technik darstellt, ist ein Verfahren zur Herstellung eines Werkstoffs mit einem Gerüst aus schalenartigen Strukturen und einem interkonnektierenden Porensystem bekannt. Bei diesem Verfahren werden deformierbare Formkörper in eine Form zur Ausbildung einer Schüttung der deformierbaren Formkörper eingefüllt und mit Überdruck beaufschlagt, die Form wird mit einem gieß-, spritz- oder schüttfähigen Material befüllt und das Material verfestigt, und die deformierbaren Formkörper werden entfernt, wobei das Material das Gerüst aus schalenartigen Strukturen mit einem interkonnektierenden Porensystem bildet.

Aus US 6,316,091 B 1 ist ein Verfahren zum Herstellen eines synthetischen Knochenersatzes bekannt, mit dem eine makroporöse synthetische Keramik mit Poren mit kontrollierten Abmessungen hergestellt werden soll, die bezüglich Anzahl und Oberfläche in einer vorbestimmten Weise verteilt sein sollen, wobei die Interkonnektion zwischen den Poren kontrolliert werden soll.

Die Erfindung ist durch die Merkmale des Implantatkörpers und seines Herstellungsverfahrens gemäß der Patentansprüche gekennzeichnet.

Die Anwendung von einem auf die formgebenden Elemente einwirkenden Unterdruck führte überraschend zu einem perfekten Ergebnis. Dabei werden die locker in ein Werkzeug geschütteten formgebenden Elemente entweder vor der Abfüllung in einem vakuumdichten System mit einem definierten Unterdruck beaufschlagt und anschliessend die gerüstbildende Masse eingesaugt und unter Einwirkung des definierten Unterdruckes bei definierter Temperatur über eine definierte Zeit aushärten gelassen, oder nach der Abfüllung, zusammen mit dem Gerüstwerkstoff mit definiertem Unterdruck beaufschlagt und gleichzeitig abgekühlt, beispielsweise über eine metallene Stellplatte:
Diese fast noch einfachere Anordnung führte zu weiter standardisierbaren Ergebnissen und beinhaltet folgende Schritte: die lockere Schüttung von formgebenden Elementen wurde mit der strukturbildenden Gerüstmasse umgossen und das geschlossene Gefäss, welches nicht vakuum - versiegelt war, wurde in einem dichten mit Unterdruck beaufschlagten Gefäss über eine bestimmte Zeit bei definierter Temperatur einem definierten Unterdruck ausgesetzt und gleichzeitig über die Stellplatte abgekühlt. Das einfache Handling dieses Vorgehens liess das Ergebnis hoch reproduzierbar werden.

Doch auch dieses Ergebnis konnte zufällig durch eine zunächst unscheinbare Veränderung, vor allem im Hinblick auf die einstellbare Porosität der Oberfläche, weiter verbessert werden:
Nahm man nämlich statt eines festen metallischen Werkzeuges eine deformierbare Silikonform, so führte dies dazu, dass das Implantat, abhängig vom angelegten Unterdruck eine durchgehende bis in die Oberfläche reichende Porosität aufwies, die durch die Höhe des Unterdruckes auf der einen Seite und den E-Modul des Werkzeuges auf der anderen Seite einstellbar war. Dieses Verfahren liess auch dann eine durchgehende Porosität erreichen, wenn die formgebenden Elemente nicht expansionsfähig, also beispielsweise nicht lufthaltig, sondern beispielsweise Zuckerkugeln waren.

Als formgebende Elemente werden bei diesem Verfahren vorzugsweise expandierbare Polystyrol-Kugeln (EPS) verwendet, beispielsweise Styropor® F414, das mit Pentan als Treibmittel aufgeschäumt ist. Beim Anlegen eines Unterdruckes dehnen sich diese Kugeln sehr schnell aus und nehmen an Volumen zu. Auf diese Weise wird die Kontaktbrücke zwischen den Kugeln breiter und bestimmt damit die Durchmesser der interkonnek-tierenden Durchtritte bis hinein in die Oberfläche; bei Verwendung eines Silikonwerkzeuges drücken sich die Kugeln in die Silikonwand und zudem zieht der Unterdruck die deformierbare Wand über die Kugeloberfläche in das Implantat hinein.

Geschäumte Werkstoffe zur Verwendung als formgebende Elemente werden bevorzugt eingesetzt, auch solche, denen Treibmittel zugesetzt sind, welche bei einer bestimmten Temperatur oder unter bestimmten Voraussetzungen aktiviert werden. Ein besonders bevorzugtes Material stellt das Styropor® F414 dar mit bevorzugten Raumgewichten des aufgeschäumten Polystyrols zwischen 17g/l und 70g/l, vorzugsweise etwa 20g/l bis 35g/l. Die Korngrössenverteilung des aufgeschäumten Materials liegt zwischen 200µm und 15mm, häufig verwandt sind die Grössen zwischen 1000µm und 3000µm.

Zur Bestimmung der Expansion und der Deformierbarkeit der einzelnen formgebenden Elemente wurden Versuche durchgeführt; um in einfacher Weise die Parameter zu bestimmen, die zur Standardisierung des Verfahrens notwendig sind. Hierfür wurden verschiedene formgebende Elemente mit verschiedenen Raumgewichten, beispielsweise verschieden geschäumte Polystyrol-Kugeln mit verschiedenen Durchmessern, in einem Zylinder mit beweglichen, vakuumdicht anliegenden Kolben bis zu einer definierten Höhe von 84,3mm aufgeschüttet und einem definierten Vakuum ausgesetzt ; aus.der Veränderung der Ausgangshöhe in Abhängigkeit vom angelegten Unterdruck wurden Grössen ermittelt, die eine anfängliche Volumenreduktion durch Entfernen der Luft zwischen den formgebenden Elementen widerspiegeln, gefolgt von einer Volumenausdehnung, die durch Aufbringen einer Kraft F, gemessen in N, zur alten Ausgangslänge einstellbar war und den Expansionsdruck der Luft in den formgebenden Elementen widergab. In Abhängigkeit von der Zeit nahm die Kraft langsam ab, was durch das Platzen der Luftblasen im Kunststoff zu erklären war. Aus diesem Phänomen wurden die definierten Zeiten ermittelt für die Beaufschlagung mit Unterdruck.

Drücke zwischen 150mbar und 800mbar, vorzugsweise um 300mbar bis 500mbar über eine Zeit von 15 Minuten einwirkend auf ein Calciumphosphat - Agar-Agar Gemisch, bei einer Temperatur des Implantates von 4-12°C zeigten besonders günstige Ergebnisse in Bezug auf die Aussenporosität und die inneren Interkonnexionen.

Als deformierbare Formen eignen sich vor allem giess- oder im Spritzgussverfahren verwendete Silikone einer Shorehärte unter 25 Shore, vorzugsweise unter 18-20 Shore. Es können aber alle plastisch oder elastisch deformierbaren Werkstoffe Verwendung finden, deren E-Modul deutlich unter dem der formgebenden Elemente liegt. Entsprechend können die Werkzeuge gegossen werden, aber auch in grossen Serien im Spritzgussverfahren hergestellt werden. Beispiele für plastisch deformierbare Werkzeuge sind Werkzeuge aus Styropor® verschiedener Dichte, Beispiele für elastisch deformierbare Werkzeuge sind die oben erwähnten Silikone, wobei auch geschäumte Silikone zur Anwendung kommen können. Der Expansionsdruck dieser Werkstoffe im Vakuum addiert sich in seiner Wirkung zu dem der formgebenden expansionsfähigen Elemente.

Als giessfähiges Material für den Gerüstwerkstoff wird vorzgsweise eine Mischung aus Hydroxylapatit (HA) oder Tricalciumphosphat (TCP) und einer Agar-Agar Lösung im Verhältnis 10g Pulver / 7ml bis zu 25ml Lösung, was einem Verhältnis von 1,4 bis 0,4 entspricht, ganz optimal sind Ansätze, bei denen das Mischungsverhältnis Pulver zu Lösung 0,45 bis 0,48 entspricht, beispielsweise 1600g HA auf 3500ml Lösung.

Je nach Zusammensetzung kann aus dem Ansatz bereits der Schrumpfungsfaktor berechnet werden: dieser liegt bei einem giessfähigen HA-Agar-Agar Gemisch, welches bei 60°C abgefüllt wird zwischen 0,95 bis 2,9, vorzugsweise zwischen 1,75- und 2,15, bei einem Verhältnis von 16g/35ml einer 1,7%igen Agar-Agar Lösung bei exakt 1,91. Mit diesen beiden Voraussetzungen, expandierbare formgebende Elemente, einer deformierbaren Silikonform und einem exakten Ansatz mit definierter Schrumpfung liessen sich sehr präzise Implantate "net shaped" sintern, ohne dass eine Nachbearbeitung notwendig wurde.

Das definitive Design multipliziert mit dem Schrumpfunssfaktor ergibt beispielsweise ein Implantatkörper in Kunststoff oder einem anderen im CAD/CAM Verfahren leicht bearbeitbaren Material, welches in einem Stammform bis zur Oberkante mit einem giessfähigen Silikon umgossen wird. Nach Aushärten des Silikons kann der formgebende Körper leicht wieder mechanisch entfern werden, die Silikonform wird am Boden mehrfach perforiert und danach mit Polystysrolkugeln der gewünschte Grösse aufgeschüttet, danach mit einem Silikondeckel mit Entlüftungslöchern verschlossen und in einer Abfüllform mit beispielsweise der Keramikmasse abgefüllt. Unmittelbar nach der Abfüllung wird das Werkzeug in toto in einem Exsiccator mit einem Unterdruck beaufschlagt und auf 4-12°C heruntergekühlt, beispielsweise über die Stellplatte. Nach Aushärten der Keramikmasse kann das Werkzeug demontiert werden und der Grünling ist leicht zu entfernen. Im Acetonbad wird nun aus dem Keramik-Styropor®-Implantat das Styropor herausgelöst, die Keramik dehydratisiert in Schritten 70/80/90 und 100%iges Aceton und anschliessend unter Kühlung schrittweise an Luft getrocknet (Kältetrocknung). Das Ergebnis wird stundenweise mit Hilfe einer Feinwaage dokumentiert, sowie kein weiterer Gewichtsverlust an Luft sich abzeichnet und die Gewicbtskurve linear gleich bleibt, wird das Implantat über 24h im Exsiccator unter Zusatz von P₂O₅ unter Vakuum 150- 250 mbar Absolutdruck getrocknet und anschliessend bei 1300°C im Sinterofen gebrannt. Das Ergebnis ist ein offenporiges, absout masshalti ges Implantat mit einer gegenüber mechanisch nachbearbeiteten Knochenersatzwerkstoff-Zylindern oder Würfeln (Draenert et.al.2001:Synthetische Knochenersatzwerkstoffe auf HA- und TCP-Basis Trauma Berufskrh 3:293-300 Heidelberg New York Berlin Tokyo: Springer) deutlich höheren Festigkeit, die durch äussere Strukturierung weiter vergrössert werden kann, beispielsweis Ringe, Einschnürungen, massive Kanten usw.

### Beispiel 1

Styropor®-Kugel von 12mm Durchmesser werden in einem zylindrischen Gefäss mit perforiertem Boden mit Bohrungen von 10-11mm im Durchmesser und einem schlüssig den Kugeln aufsitzenden fixierbaren Deckel mit denselben Bohrungen in einen Becher getaucht und intrudiert, der mit 90°C heissem Wachs gefüllt und hierfür aus dem Heizofen genommen wird. Der Behälter mit den Styropor®-Kugeln passt dabei mit seinem Aussendurchmesser schlüssig in das zylindrische Wachsgefäss, welches einen demontierbaren Boden aufweist. Nach Aushärten des Wachses bei Zimmertemperatur, wird der Boden des Wachsgefässes demontiert und der Styropor®-Behälter herausgedrückt. Der Styropor®-Behälter wird ebenfalls von seinem Boden und dem Deckel befreit und der Wachs-Styropor®-Zylinder herausgeschoben und in einem Acetonbad von dem Styropor® befreit. Nach Trocknung an Luft wird das so entstandene durchgehend interkonnektierend poröse Wachsgerüst in eine dieses schlüssig aufnehmendes Abfüllgefäss eingeschoben. Das Abfüllgefäss weißt einen Einfüllstutzen mit Perforationen am Boden des Gefässes auf und kann ausserdem ein Sieb aufnehmen, welches zum Einsaugen kleinerer Styropor®-Kugeln benötigt wird, ausserdem ist es mit einem Deckel versehen, welcher Entlüftungsbohrungen aufweist, beispielsweise mit einem Durchmesser von 1,5 - 2,5mm. In das Wachsgerüst werden Styropor®-Kugeln mit einem Durchmesser von beispielsweise 600-1200µm eingesaugt, wobei im Abfüllstützen ein Sieb mit einer Maschenweit von 400µm vorgeschalten wird. Das Hohlraumsystem des Wachsgerüstes füllt sich vollständig mit den kleineren Styropor®-Kugeln auf und wird danach mit einem Deckel verschlossen. Das Gefüss wird nun mit einer Keramikmasse abgefüllt, wobei über den Abfüllstutzen die Masse über die Belüftungslöcher eingesaugt oder ohne grossen Druck einfach über den Stutzen aufgefüllt wird, Tritt die Keramikmasse aus den Entlüftungslöchem des Deckels aus, ist die Form gefüllt und wird nun mitsamt dem Werkzeug in einen Exsiccator gestellt und über 15 Minuten mit einem Unterdruck von 500-600mbar beaufschlagt und während dieser Zeit über die metallene Stellplatte abgekühlt. Der so ausgehärtete Wachs-Keramik-Styropor®-Block wird anschliessend im Acetonbad vom Styropor® befreit, an Luft getrocknet und mit dem Wachs im Ofen bei 1300°C gesintert. Das Ergebnis sind vollständig isolierte, aussen durchgehend poröse und im Innern vollstandig interkonnektierend gekammerte Kugeln mit einem Durchmesser um 6mm. Aufgrund der Schrumpfung des Matrixmaterial kommt es zur Ausbildung vollständig separierter Kugeln.

### Beispiel 2

Statt einzelner gekammerter Kugeln kann ein interessanter Werkstoff gefertigt werden, der einem Negativabdruck der Knochenmarkswaben entspricht und ein Kugelkonglomerat mit Zwischenräumen zwischen den gekammerten Kugeln für das Einwachsen von Knochen-bälkchen und breiten Verbindungsbrücken, die den Kontaktstellen der Kugeln entsprechen:
Die Schritte entsprechen dem Beispiel 1, jedoch wird das Wachs-Styropor®-Gerüst während des Aushärtens in einem Exsiccator einem definierten Unterdruck von beispielsweise 600m bar ausgesetzt. Die daraufhin erfolgende Expansion der Styropor®-Kugeln führt zu verbreiterten Brücken und dickeren, Verbindungsarmen zwischen den Kugeln. Nach Aushärten des Wachs-Styropor®-Gerüstes erfolgt das Herauslösen des Styropor®. Die nachfolgenden Schritte entsprechen dem Beispiel 1 mit Einsaugen von kleineren Styropor®-Kugeln, Auffüllen mit einer Keramikmasse, Nachevakuieren unter Abkühlen und
anschliessendem Herauslösen der formgebenden Elemente im Aceton; danach erfolgt im Gegensatz zum Beispiel 1 der Kälte-Trocknungsschritt: in Schritten von jeweils 2h wird über 70/80/90 und 3x 100 %iges Aceton in Stufen in der Kühltruhe an Luft getrocknet, bei 4-12°C aufsteigend in beispielsweise 4 Schritten von jeweils 3 Stunden bis zur Zimmertemperatur und anschliessender Dehydratation im Exsiccator unter Zuhilfenahme von P₂O₅ und einem Vakuum um 150mbar Absolutdruck über 24h. Bei einem solchen Vorgehen bleiben breite Brücken zwischen den gekammerten Kugeln bestehen und das Wachsgerüst kann mitsamt dem Keramikinlet im Ofen gebrannt werden bei beispielsweise 1300°C; das Wachs kann auch vorher im Heizofen abgeschmolzen werden bei 90°C, Voraussetzug hierfür ist, dass die Keramikmasse bereits luftgetrocknet ist. Das Ergebnis ist ein perfektes Gerüst aus zusammenhängenden im Inneren perfekt interkonnektierend gekammerten Kugeln mit durchgehend poröser Oberfläche und einer erstaunlich hohen Kompressionsfestigkeit. Diese liegt je nach Kugelgrösse bei 4-12 MPa.

### Beispiel 3

Zur Herstellung eines masshaltigen Würfels mit einer Kantenlänge von 15mm und einer offenen Porosität über alle Flächen. wird bei einer Matrixmasse von 16/35 HA/-Agar-Agar Suspension 1,7%ig ein Schrumpfungsfaktor von 1,91 berechnet und im CAD CAM Verfahren ein Würfel aus POM mit einer Kantenlänge von 28,65mm hergestellt. Da der Würfel eine hohe Festigkeit von 4-6 MPa erreichen soll, werden die Kanten nicht abgerundet. Der Würfel wird in ein Formwerkzeug gestellt und bis zu seiner Oberkante mit einem selbsthärtenden Silikon ausgegossen. Nach 24h wird der Würfel mechanisch entfernt und das Silikonwerkzeug in ein Abfüllwerkzeug eingebracht, dessen Boden innen aus einem perforierten Silikonboden besteht und nach Auffüllen mit 1200µm messenden Styropor®-Kugeln mit einem Silikondeckel mit Entlüftungslöchern schlüssig abgedeckt. Nachdem das Werkzeug mit einem Schraubdeckel mit Entlüftungslöchern verschlossen wurde, wird es mit einer Keramimasse aufgefüllt; anschliesend erfolgt im Exsiccator auf einer metallenen und kühlbaren Stellplatte die Beaufschlagung mit Unterdruck von 500mbar über 15 Minuten bei gleichzeitiger Abkühlung. Danach wird das Werkzeug demontiert und der Keramik-Styropor®-Würfel vorsichtig mechanisch herausgenommen und im Acetonbad von den formgebenden Elementen befreit. Zur rissfreien Trocknung wird die Kältedehydratisierung angeschlossen wie in den Beispielen 1 und 2 beschrieben und anschliessend der Würfel im Ofen bei beispielsweise 1300°C gesintert. Das Ergebnis ist ein masshaltiger sehr kompressionsstabiler Würfel mit offener Porosität über alle Flächen und einer durchgehend interkonnektierenden Porosität der inneren Struktur und verstärkten Kanten aus kompakter Keramik.

Die Erfindung wird nachstehend anhand der Figuren noch näher erläutert.

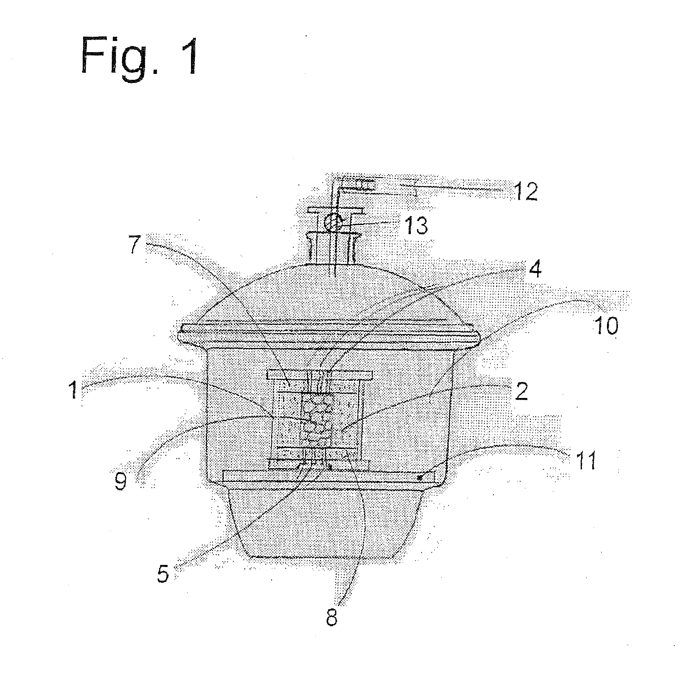

In Figur 1 ist eine einfache Vakuumkammer beschrieben in Form eines Exisccators 10 mit Belüftungsventil 13 und Hahn zum Anschluss eines Schlauches zur Vakuumpumpe 12 und einer metallenen Kühlplatte 11 mit einem auf der Kühlplatte stehenden Abfüllwerkzeug 1 mit einem schlüssig aufgenommenen deformierbaren Werkzeug in drei Teilen, einem Korpus 2, einem Deckel 7 mit Perforationen 4 und einem Boden 8 mit Perforationen 5 zum Auffüllen beispielsweise mit einer keramischen Masse.

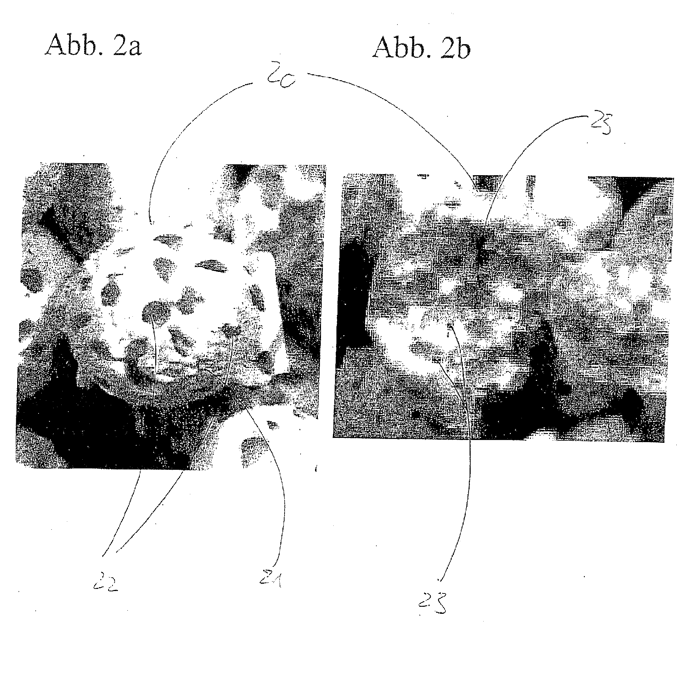

In Figuren 2a und 2b sind erfindungsgemäße keramische Implantate 20 dargestellt, gemäß Figur 2a in der Aufsicht und in Figur 2b als Bruch. Die Aufsicht auf die Kugelschale lässt die offene Porosität 22 erkennen und die raue Schale 21. Im Bruch ist die interkonnektierende Porenstruktur 23 dargestellt.

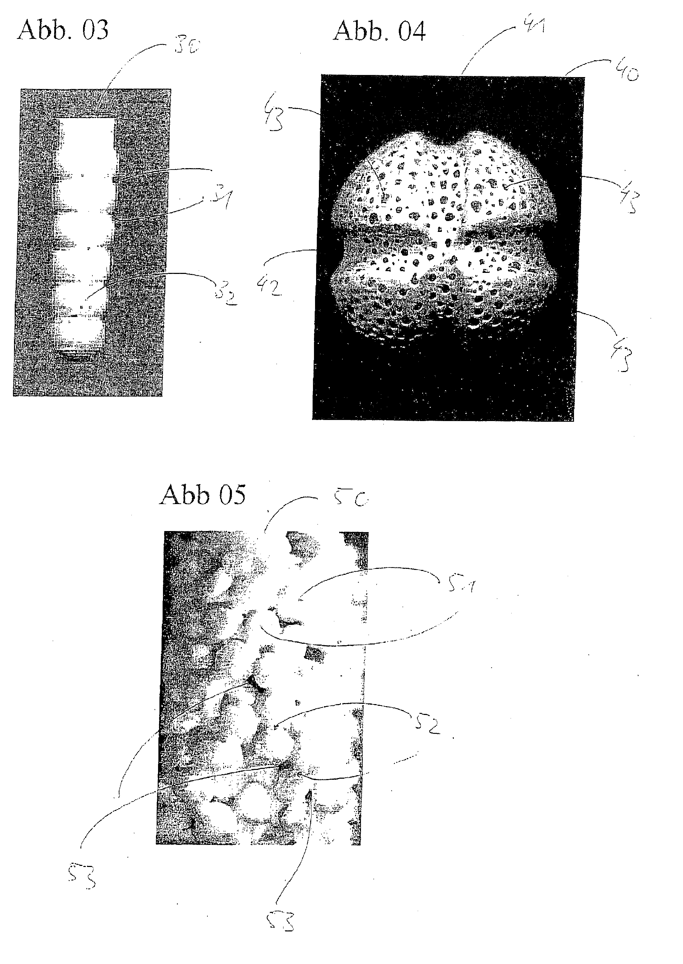

In Figur 3 ist ein erfindungsgemäßes Implantat dargestellt als Knochendübel 30, beispielsweise zur Refixation eines Bandes beim Kreuzbandersatz über die Aussenseite des Dübels, welches horizontale Einschnürungen 31 aufweist, die vorteilhaft jedoch auch schraubenförmig angeordnet sein können, und welches über die gesamte Oberfläche eine von Poren 32 unterbrochene äussere Schale aufweist.

In Figur 4 ist ein erfindungsgemäßes leichtes Keramikimplantat 40 dargestellt, net shaped gesintert, mit einer offenen Porosität über die gesamte Oberfläche 43, welches sich kreuzende Furchen oder Rinnen 41, 42 aufweist, in denen die Augenmuskeln vernäht werden können, welches als Teil eines künstlichen Auges verwendet wird.

Die Figur 5 zeigt ein Kugelkonglomerat 50, welches aus soliden Kugeln 51 besteht, welches erfindungsgemäß jedoch auch aus Elementen der Figuren 2a und 2b bestehen kann und sich durch breite Brücken 52 zwischen den Kugelelementen und regelmässige Zwischenkugelräume 53 auszeichnet.

## Patentansprüche

1. Verfahren zur Herstellung eines zylinderförmigen gekammerten Implantatkörpers, bestehend aus einer äusseren Schale (21) und einer inneren Struktur, wobei die aussere Schale eine Oberfläche aufweist, die über ihre Gesamt- oder Teilfläche von bei der Herstellung Durchmesser einstellbaren Poren durchbrochen ist, und wobei die innere Struktur aus einem Konglomerat aus von Kugelschalen umgebenen Hohlräumen besteht, wobei die Kugelschalen ein Schalengerüst bilden, das in direktem Zusammenhang mit der äusseren Schale steht, und wobei die Hohlräume ein zusammenhängendes Hohlraumsystem (23) bilden, dessen Hohlräume bei der Herstellung im Durchmesser einstellbar und durchgehend interkonnektierend verbunden sind, wobei das Hohlraumsystem der inneren Struktur durch die äussere Schale nach außen geöffnet ist, wobei das Verfahren die folgenden Verfahrensschritte aufweist :
Herstellen der äußeren Form des Implantatkörpers unter Berücksichtigung des Schrumpfungsfaktors des Werkstoffes des Schalengerüstes und der äußeren Schale durch Füllen eines Werkzeuges mittels Schüttung unter Unterdruck expandierbarer formgebender Elemente, wobei das Werkzeug eine oder mehrere Belüftungsöffnungen aufweist,
Befüllen des Werkzeugs mit einer das Schalengerüst und die äußere Schale des Implantatkörpers bildenden Masse,
Beaufschlagen des Werkzeugs mit einem Unterdruck in einer geschlossenen Kammer,
wobei das Beaufschlagen vor oder nach dem Befüllen des Werkzeugs mit der das Schalengerüst und die äußere Schale bildenden Masse erfolgt,
Entfernen der formgebenden Elemente,
Trocknen, Dehydratisieren und Brennen des nach Entfernen der formgebenden Elemente gebildeten Implantatkörpers.

2. Verfahren nach Anspruch 1, wobei nach dem Entfernen der formgebenden Elemente das entstandene Hohlraumsystem mit kleineren formgebenden Elementen aufgefüllt und das verbleibende Hohlraumsystem um die kleineren formgebenden Elemente anschließend mit einem fließfähigen Werkstoff aufgefüllt wird, welcher im fertigen Implantatkörper das Schalengerüst und die äußere Schale bildet, und wobei die kleineren formgebenden Elemente nach dem Festigen des fließfähigen Werkstoffs entfernt werden.

3. Verfahren nach Anspruch 1 oder 2, wobei lufthaltige formgebende Elemente verwendet werden.

4. Verfahren nach einem der Ansprüche 1 bis 3, wobei die formgebenden Elemente aus expandierbarem Polystyrol (EPS) oder Styropor® bestehen und sich bei der Beaufschlagung mit Unterdruck ausdehnen können, so dass sich breitere Kontaktbrücken zwischen formgebenden Elementen ausbilden, wodurch die Interkonnexion und Oberflächenporosität des fertigen Implantatkörpers einstellbar ist.

5. Verfahren nach einem der Ansprüche 1 bis 4, wobei das Werkzeug aus Polystyrol oder Silikon besteht und/oder wobei die Unterdruckkammer temperiert ist und bei definierter Temperatur ein definierter Unterdruck angelegt wird, um die Ausdehnung der formgebenden Elemente und damit die Hohlräume des zusammenhängenden Hohlraumsystems, deren Interkonnexion und die Größe der Poren der äußeren Schale zu steuern.

6. Zylinderförmiger gekammerter Implantatkörper, hergestellt mit dem Verfahren nach einem der Ansprüche 1 bis 5.

7. Implantatkörper nach Anspruch 6, wobei die äussere Schale strukturiert ist in Form von einer oder mehreren zirkulären oder radiären, longitudinalen, schrägen oder spiralförmigen, parallel oder sich kreuzend verlaufenden Einschnürungen, Furchen und/oder Wülsten oder in Form einzelner oder mehrerer Erhebungen, Eindellungen oder Zapfen.

8. Implantatkörper nach Anspruch 6 oder 7, wobei die innere Struktur einem schalenförmigen Schwamm- oder Spongiosaknochen entspricht.

9. Implantatkörper nach einem der Ansprüche 6 bis 8, wobei mindestens eine Pore der äusseren Schale in ihrem Durchmesser kleiner ist als der Durchmesser eines mit ihr in Verbindung stehenden Hohlraumes der inneren Struktur, wobei das Verhältnis der Durchmesser vorzugsweise 1:5 bis 1:1,5, im Mittel vorzugsweise 1:2 bis 1:3 beträgt.

10. Implantatkörper nach einem der Ansprüche 6 bis 9, wobei das Konglomerat der inneren Struktur aus Einzelimplantaten ausgebildet ist, die mit einstellbar breiten Brücken miteinander verbunden sind und das zusammenhängende Hohlraumsystem umschließen, wodurch die Einzelimplantate in der Weise von Knochen durchwachsen werden können, dass ein physiologisches Schwammknochengerüst entsteht, dessen Durchtritte durch die Verbindungsbrücken der Einzelimplantate vorgegeben sind.

11. Implantatkörper nach einem der Ansprüche 6 bis 10, wobei der Werkstoff des Schalengerüsts ein Calciumphosphat, wie Tri-Calciumphosphat oder Tetra-Calciumphosphat, ein Hydroxylapatit, ein Calciumcarbonat, eine Calciumsufatverbindung, eine Aluminiumoxidkeramik, ein Kollagen, eine Polyaminosäure oder ein anderes resorbierbares Polymer ist.

12. Implantatkörper nach einem der Ansprüche 6 bis 1.1, wobei das Hohlraumsystem ganz oder teilweise mit einem resorbierbaren oder nicht resorbierbaren organischen oder anorganischen Füllwerkstoff mit dämpfenden oder versteifenden Eigenschaften aufgefüllt oder beschichtet ist, wobei der Füllwerkstoff sich auf das Innere des Implantatkörpers beschränkt oder diesen vollständig ausfüllt, jedoch die äussere Schale nicht überragt, oder wobei der Füllwerkstoff regelmäßig oder unregelmäßig aus den Poren der äusseren Schale hervorquillt und tropfenförmige und/oder kugelförmige oder ähnlich konfigurierte Erhebungen über die äussere Schale bildet, die gleichmäßig oder ungleichmäßig über die Oberfläche der äusseren Schale verteilt sind, wodurch Mikrobewegungen mehrerer gleicher oder ähnlicher Implantatkörper, beispielsweise einer Schüttung, ganz oder nahezu ganz verhindert werden.

13. Implantatkörper nach Anspruch 12, wobei als Füllwerkstoff eine resorbierbares Polymer, beispielsweise eine Polyaminosäure, ein Polylactid, Polyglactin oder Glycosid, ein Polyacetal, ein Polysäureamid oder Polyester, oder ein nicht resorbierbares Polymer, wie Polymethylmethacrylat oder Agarose oder ein Gemisch aus Agarose und einem Calciumphosphat oder Calciumcarbonat verwendet wird.

14. Implantatkörper nach einem der Ansprüche 6 bis 13, wobei in standardisierter Weise weite und engere Hohlraumsysteme ausgebildet sind, die zusammenhängend oder unterbrochen, einzeln oder in Gruppen oder entlang einer Achse angeordnet sind und unterschiedlich weite Interkonnexionen aufweisen, wodurch sich die Hohlräume leichter oder schwerer mit Füllwerkstoffen ausfüllen lassen.

15. Implantatkörper nach einem der Ansprüche 6 bis 14, wobei in dem Hohlraumsystem verschieden große Hohlräume mit einer Wandung aus einem Werkstoff und einem interkonnektierenden Schalengerüst aus einem anderen resorbierbaren oder nicht resorbierbaren Werkstoff aufgebaut sind, beispielsweise ein großer Hohlraum mit einer schalenförmigen Wandung aus Hydroxylapatit und mit einem Schalengerüst aus einem leicht resorbierbaren Tricalciumphosphat.

## Claims

1. A method for manufacturing a cylindrical chambered implant body consisting of an outer shell (21) and an inner structure, wherein said outer shell has a surface which is perforated over its entire or partial area by pores whose diameter is adjustable during manufacturing, and wherein said inner structure is formed from a conglomerate of cavities surrounded by sphere-shaped shells, wherein the sphere-shaped shells form a shell frame having a direct connection to the outer shell, and wherein the cavities form an interrelated cavity system (23), the diameter of the cavities of said cavity system being adjustable during manufacturing and said cavities being continuously interconnected, wherein the cavity system of the inner structure is opened through the outer shell to the outside,
wherein said method comprises the following method steps:
preparing the outer shape of the implant body considering the shrinking factor of the material of the shell frame and the outer shell by filling a mold with shaping elements being expandable under underpressure, wherein the mold comprises one or multiple openings for venting;
filling the mold with a material forming the shell frame and the outer shell of the implant body;
applying underpressure to the mold in a closed chamber, wherein the underpressure is applied prior to or after the filling of the mold with the material forming the shell frame and the outer shell;
removing the shaping elements;
drying, dehydrating and calcinating the implant body formed after the removal of the shaping elements.

2. The method according to claim 1, wherein the cavity system formed after the removal of the shaping elements is filled with smaller shaping elements and the remaining cavity system around the smaller shaping elements is subsequently filled with a free-flowing material which forms the shell frame and the outer shell in the finished implant body and wherein the smaller shaping elements are removed after solidification of the free-flowing material.

3. The method according to claim 1 or 2, wherein shaping elements containing air are used.

4. The method according to any one of claims 1 to 3, wherein the shaping elements consist of expandable polystyrene (EPS) or Styropor® and can expand upon the application of underpressure so that wider contact bridges form between shaping elements, whereby the interconnection and surface porosity of the finished implant body are adjustable.

5. The method according to any one of claims 1 to 4, wherein the mold consists of polystyrene or silicone and/or wherein the underpressure chamber is tempered and a defined underpressure is applied at a defined temperature in order to thus control the expansion of the shaping elements and thus the cavities of the interrelated cavity system, the interconnection of the cavities and the size of the pores of the outer shell.

6. A cylindrical chambered implant body manufactured with the method according to any one of claims 1 to 5.

7. The implant body according to claim 6, wherein the outer shell is structured in the shape of circular or radial, longitudinal, oblique or helical, parallel or crossing contractions, channels and/or bulges, singular or in a plurality thereof, or in the shape of single or plural elevations, recesses or pins.

8. The implant body according to claim 6 or 7, wherein the inner structure corresponds to a bowl-shaped sponge bone or cancellous bone.

9. The implant body according to any one of claims 6 to 8, wherein at least one pore of the outer shell has a diameter smaller than the diameter of a cavity of the inner structure connected therewith, wherein the ratio of the diameters is preferably 1:5 to 1:1.5, on average preferably 1:2 to 1:3.

10. The implant body according to any one of claims 6 to 9, wherein the conglomerate of the inner structure is formed of individual implants which are connected to each other via bridges having adjustable widths and encompass the interrelated cavity system, which enables bone to grow through individual implants in a manner that a physiological sponge bone frame is generated, the passages of which are determined by the connecting bridges of the individual implants.

11. The implant body according to any one of claims 6 to 10, wherein the shell frame material is a calcium phosphate, such as tricalcium phosphate or tetracalcium phosphate, a hydroxy apatite, a calcium carbonate or calcium sulfate composition, an aluminum oxide ceramic, a collagen, a polyamino acid or any other resorbable polymer.

12. The implant body according to any one of claims 6 to 11, wherein the cavity system is filled or coated completely or partially with a resorbable or non-resorbable organic or inorganic filler material having dampening or reinforcing properties, wherein the filler material is restricted to the inside of the implant body or completely fills the implant body, but does not extend beyond the outer shell, or wherein the filler material regularly or irregularly wells out of the pores of the outer shell to form drop-shaped and/or sphere-shaped or similarly shaped elevations over the outer shell which are regularly or irregularly distributed over the surface of the outer shell, whereby micromovements of plural identical or similar implant bodies, for example in a fill, are completely or almost completely prevented.

13. The implant body according to claim 12, wherein a resorbable polymer, e.g. a polyamino acid, a polylactide, polyglactin or glycoside, polyacetal, polyacid amide or polyester, or a non-resorbable polymer like polymethyl methacrylate or agarose or a mixture of agarose and a calcium phosphate or calcium carbonate is used as the filler material.

14. The implant body according to any one of claims 6 to 13, wherein wide and narrow cavity systems are formed in a standardized manner which are arranged in an interrelated or interrupted manner, individually or in groups or along an axis and comprise interconnections of different widths, whereby the cavities can be filled more or less easily with filler materials.

15. The implant body according to any one of claims 6 to 14, wherein cavities of different sizes with a wall of a material and an interconnecting shell frame of another resorbable or non-resorbable material are formed in the cavity system, for example a large cavity having a shell-shaped wall of hydroxy apatite and having a shell frame of an easily resorbable tricalcium phosphate.

## Revendications

1. Procédé de fabrication d'un corps d'implant compartimenté cylindrique, constitué d'une coque externe (21) et d'une structure interne, dans lequel la coque externe a une surface perforée sur toute sa surface ou une partie de sa surface par des pores ajustables en diamètre lors de la fabrication, et dans lequel la structure interne est constituée d'un conglomérat de cavités entourées de coques sphériques, lesdites coques sphériques formant une ossature de coques en liaison directe avec la coque externe, et dans lequel les cavités forment un système cohérent de cavités (23), lesdites cavités étant ajustables en diamètre lors de la fabrication et interconnectées entre elles de manière continue, le système de cavités de la structure interne étant ouvert vers l'extérieur par la coque externe, ledit procédé comprenant les étapes de procédé suivantes :
fabrication de la forme externe du corps d'implant, en tenant compte du facteur de contraction du matériau de l'ossature de coques et de la coque externe en remplissant un moule avec des éléments de formage expansibles par dépression, le moule comportant une ou plusieurs ouvertures d'aération,
remplissage du moule avec une masse formant l'ossature de coques et la coque externe du corps d'implant,
exposition du moule d'une dépression dans un compartiment clos, l'exposition ayant lieu avant ou après le remplissage du moule avec la masse formant l'ossature de coques et la coque externe,
retrait des éléments de formage,
séchage, déshydratation et cuisson du corps d'implant formé après le retrait des éléments de formage.

2. Procédé selon la revendication 1, dans lequel, après retrait des éléments de formage, le système de cavités ainsi formé est rempli avec des éléments de formage plus petits et le système de cavités restant est ensuite rempli d'un matériau à écoulement libre autour des éléments de formage plus petits, qui forme l'ossature de coques et la coque externe dans le corps d'implant fini, et dans lequel les éléments de formage plus petits sont retirés après solidification du matériau à écoulement libre.

3. Procédé selon la revendication 1 ou 2, dans lequel des éléments de formage contenant de l'air sont utilisés.

4. Procédé selon l'une des revendications 1 à 3, dans lequel les éléments de formage sont constitués de polystyrène expansible (PSE) où de Styropor® et peuvent s'étendre lors de l'exposition d'une dépression, pour que des ponts de contact larges se forment entre des éléments de formage, afin que l'interconnexion et la porosité de surface du corps d'implant fini soient réglables.

5. Procédé selon l'une des revendications 1 à 4, dans lequel le moule consiste de polystyrène ou de silicone et/ou dans lequel la chambre de dépression est tempérée et une dépression définie est créée à une température définie, pour régler l'expansion des éléments de formage et ainsi les cavités du système cohérent de cavités, leur interconnexion et la taille des pores de la coque externe.

6. Corps d'implant compartimenté cylindrique fabriqué selon le procédé selon l'une des revendications 1 à 5.

7. Corps d'implant selon la revendication 6, dans lequel la coque externe est structurée sous la forme d'étranglements, de gorges et/ou de boudins circulaires ou radiaux, longitudinaux, obliques ou hélicoïdaux, parallèles entre eux ou se croisant, isolés ou en nombre, ou sous la forme de reliefs, d'empreintes, ou tenons, isolés ou en nombre.

8. Corps d'implant selon la revendication 6 ou 7, dans lequel la nature de la structure interne correspond à un os spongieux en forme de coque.

9. Corps d'implant selon l'une des revendications 6 à 8, dans lequel au moins un pore de la coque externe présente un diamètre inférieur au diamètre d'une cavité de la structure interne qui lui est reliée, le rapport des diamètres étant compris de préférence entre 1:5 et 1:1,5, en moyenne de préférence entre 1:2 et 1:3.

10. Corps d'implant selon l'une des revendications 6 à 9, dans lequel le conglomérat de la structure interne est formé d'implants individuels qui sont reliés entre eux par des ponts de largeur ajustable et qui entourent le système cohérent de cavités, lequel permet aux implants individuels d'être entregreffés d'os, pour que une ossature spongieuse physiologique se forme, dont les passages sont définis par les ponts de liaison des implants individuels.

11. Corps d'implant selon l'une des revendications 6 à 10, dans lequel le matériau de l'ossature de coques est un phosphate de calcium comme le tricalcium phosphate ou le tétracalcium phosphate, une hydroxylapatite, un carbonate de calcium, un composé de sulfate de calcium, une céramique d'oxyde d'aluminium, un collagène, un acide polyaminé ou un autre polymère résorbable.

12. Corps d'implant selon l'une des revendications 6 à 11, dans lequel le système de cavités est totalement ou partiellement rempli ou revêtu d'un matériau de charge organique ou inorganique, résorbable ou non résorbable, ayant des propriétés d'amortissement ou de renforcement, dans lequel le matériau de charge reste limitée à l'intérieur du corps d'implant ou remplit totalement celui-ci mais sans dépasser de la coque externe, ou dans lequel le matériau de charge sort régulièrement ou irrégulièrement des pores de la coque externe et forme des reliefs en forme de gouttes et/ou sphériques ou de configuration analogue sur la coque externe, répartis de manière homogène ou inhomogène à la surface de la coque externe, moyennant quoi des micro-déplacements de plusieurs corps d'implant identiques ou similaires, par exemple dans une charge, sont empêchés totalement ou presque totalement.

13. Corps d'implant selon la revendication 12, dans lequel le matériau de charge utilisé est un polymère résorbable, tel qu'un acide polyaminé, un polylactide, polyglactine ou glycoside, polyacétale, polyacide amique ou polyester, ou un polymère non résorbable, tel que polyméthacrylate de méthyle ou agarose ou un mélange d'agarose et d'un phosphate de calcium ou carbonate de calcium.

14. Corps d'implant selon l'une des revendications 6 à 13, dans lequel des systèmes de cavités larges et étroits sont réalisés de manière standardisée, lesquels sont disposés de manière cohérente ou interrompue, isolément ou en groupes ou le long d'un axe, et présentent des interconnexions de grandeurs différenciées, de façon que les cavités se laissent ainsi remplir plus facilement ou plus difficilement par des matériaux de charge.

15. Corps d'implant selon l'une des revendications 6 à 14, dans lequel des cavités de différente taille avec une paroi d'un matériau et une ossature de coques interconnectant d'un autre matériau résorbable ou non résorbable sont formés dans le système de cavités, par exemple une grande cavité avec une paroi d'hydroxylapatite en forme de coque et avec une ossature de coques d'un tricalcium phosphate facilement résorbable.
